# FASCICULE DE BREVET EUROPEEN

(11) **EP 0 494 005 B1**
(45) Date de publication et mention de la délivrance du brevet: **05.02.1997**
(21) Numéro de dépôt: 91403479.8
(22) Date de dépôt: 20.12.1991
(51) Int. Cl.: A61K 31/505, A61K 31/53, A61K 31/445

(54) **Agent à plusieurs composants ou kit de préparation de la forme sulfoconjuguée de composés pyridino-,pyrimidino- ou triazino-N-oxyde et procédé de mise en oeuvre**
Mehrkomponentenwirkstoff oder Kit zur Herstellung von sulfokonjugierten Formen von Pyridino-, Pyrimidino- oder Triazino-N-Oxydeverbindungen und Verfahren zu ihrer Verwendung
Multicomponent agent or kit for the preparation of the sulphoconjugated forms of pyridino-, pyrimidino- or triazino-N-oxyde compounds and method of application thereof

(30) Priorité: 28.12.1990 FR 9016504
(43) Date de publication de la demande: 08.07.1992
(73) Titulaire: L'OREAL, 75008 Paris (FR)
(72) Inventeur: Gaetani, Quintino, F-93270 Sevran (FR); Rougier, André, F-93340 Le Raincy (FR); Duranton, Albert, F-75018 Paris (FR); Hocquaux, Michel, F-75012 Paris (FR)
(74) Mandataire: Dossmann, Gérard

(56) Documents cités:
- WO-A-86/04231
- GB-A- 2 071 092
- J. MED. CHEM., vol. 26, no. 12, décembre 1983, pages 1791-1793, US; J.M. McCALL et al.: "Pyrimidine and triazine 3-oxide sulfates: A new family of vasodilators"
- PATENT ABSTRACTS OF JAPAN, vol. 11, no. 398 (C-466)[2845], 25 décembre 1987; & JP-A-62 158 204

## Description

La présente invention concerne un agent à plusieurs composants ou kit de préparation de la forme sulfo-conjuguée de composés pyridino-, pyrimidino- ou triazino-N-oxyde ainsi qu'un procédé pour sa mise en oeuvre.

Certains dérivés de sulfate de pyridines, pyrimidines et de triazines ont été décrits dans US-A-4.287.338, notamment pour le traitement systémique de l'hypertension. L'application topique des sels internes de sulfate de pyrimidines à la stimulation de la repousse des cheveux fait l'objet de la demande de brevet WO 86-O4231. Ce document décrit en particulier l'utilisation du sel interne de l'hydroxyde du 2,4-diamino 6-pipéridinyl 3-sulfoxy pyrimidinium, connu également sous la dénomination "sulfate de minoxidil".

Ce composé présente l'inconvénient d'être difficilement accessible par synthèse et d'être peu stable au stockage en milieu aqueux ou hydro-alcoolique. Pour garantir sa bonne conservation et empêcher sa décomposition, il est nécessaire de le stocker à -15°C. De plus, il s'est avéré difficile de préparer, isoler et purifier un sulfate chimiquement pur, d'une part, et, d'autre part, une solution de "sulfate de minoxidil" en milieu aqueux présente une durée de 1/2 vie de 48 heures. Il en résulte que le produit n'est pas industriellement applicable, et que du fait des durées de vie de cet ordre de grandeur, ce composé préparé par synthèse est difficilement compatible, notamment avec les utilisations thérapeutiques.

D'autres composés du type pyridino-, pyrimidino- ou triazino-N-oxyde dans la forme sulfo-conjuguée, présentent les mêmes inconvénients et peuvent avoir une durée de demi-vie encore plus courte.

La présente invention concerne des agents à plusieurs composants ou "kit" de préparation de la forme sulfo-conjuguée de composés pyridino-, pyrimidino- ou triazino-N-oxyde, destinés à être mis en contact avant l'emploi, caractérisés en ce qu'ils comportent, sous forme séparée, au moins :
a) un composant contenant dans un milieu alcoolique, hydroalcoolique ou aqueux, au moins un composé de formule (I) : 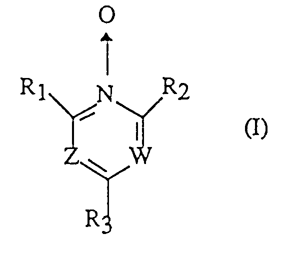 dans laquelle :
   R₁ et R₂ désignent alkyle inférieur en C₁ à C₈ ou un groupement amino;
   W et Z désignent chacun un groupement -CH= ou -N=;
   R₃ désigne un groupement :
      -S-R₄,-O-R₄, 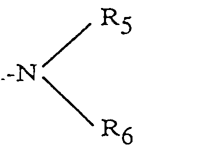 ou -H; et
   R₄ désigne un groupement alkyle linéaire ou ramifié en C₁ à C₁₈ non substitué ou substitué par un ou plusieurs atomes d'halogène; alcényle en C₁ à C₁₈; cycloalkyle en C₃ à C₇; aryle ou aralkyle;
   R₅ et R₆ désignent, indépendamment l'un de l'autre, un atome d'hydrogène ou un groupement tel que défini pour R₄; ou R₅ et R₆ forment avec l'azote auquel ils sont reliés, un reste hétérocyclique choisi dans le groupe constitué par les groupements pipéridino, pyrrolidinyle, morpholino, 2,4,4-triméthylazétidinyle, 2,3,4-triméthylazétidinyle, 2-méthylpyrrolidinyle, 3-butylpyrrolidinyle, 2-isohexylpyrrolidinyle, 2,3-diméthylpyrrolidinyle, 2,2-diméthylpyrrolidinyle, 2,5-diéthylpyrrolidinyle, 3-tert-butylpyrrolidinyle, 2,3,5-triméthylpyrrolidinyle, 3,4-dioctylpyrrolidinyle, 2-méthylpipéridino, 3-méthylpipéridino, 4-méthylpipéridino, 3-isopropylpipéridino, 4-tert-butylpipéridino, 2-méthyl-5-éthylpipéridino, 3,5-dipentylpipéridino, 2,4,6-triméthylpipéridino, 2,6-diméthylpipéridino, 2,6-diméthyl-4-octylpipéridino, 2,3,5-triéthylpipéridino, 2-éthylhexahydroazepinyle, 4-tert-butylhexahydroazépinyle, 3-heptylhexahydroazépinyle, 2,4-diméthylhexahydroazépinyle, 3,3-diméthylhexahydroazépinyle, 2,4,6-tripropylhexahydroazépinyle, 2-méthylheptaméthylène-imino, 5-butylheptaméthylène-imino, 2,4-diisopropylheptaméthylène-imino, 3,3-diéthylheptaméthylène-imino, 2,5,8-triméthylheptaméthylène-imino, 3-méthyloctaméthylène-imino, 2,9-diéthyloctaméthylène-imino, 4-isooctyloctaméthylène-imino, 2-éthylmorpholino, 2-méthyl-5-éthylmorpholino, 3,3-diméthylmorpholino, 2,6-di-ter-butylmorpholino, 4-méthylpipérazinyle, 4-isopropylpipérazinyle, 2-méthylaziridinyle, 2-éthylaziridinyle, 2-butylaziridinyle, 2,3-diméthylaziridinyle, 2,2-diméthylaziridinyle, 2-méthylazétidinyle, 3-méthylazétidinyle, 2-octylazétidinyle, 2,2-diméthylazétidinyle, 3,3-diéthylazétidinyle; ou leurs sels d'addition par des acides; et
b) un composant comportant au moins un agent de sulfatation sous forme solide.

Les composants a) et b) peuvent être sous forme solide, l'agent ou le "kit" comporte alors un troisième composant c) formé par un milieu liquide, véhicule susceptible de dissoudre les composants a) et b) et de former avec les composants a) et b), en fin de réaction, un milieu physiologiquement acceptable.

Le composant c) est destiné à être additionné au composant a) ou b) avant la mise en contact avec b) ou a) respectivement ou aux composants a) et b) après leur mise en contact.

La présente invention résoud donc les problèmes de difficulté de synthèse et de stabilité des formes sulfo-conjuguées qui peuvent être ainsi préparées juste avant leur emploi et, comme cela apparaîtra dans les exemples, garder une activité prolongée de telie façon qu'après leur préparation, elles peuvent être utilisées pendant une semaine en application quotidienne.

Le composé de formule (I) est mis en contact avec un générateur de SO₃ dans un milieu réactionnel.

Le milieu réactionnel résulte, soit du mélange de a) et b), soit du mélange de a) et c) puis b), du mélange de b) et c) puis a) ou du mélange de a) et b) puis c).

L'application ou l'administration peuvent être faites en vue d'un traitement de l'hypertension, de l'alopécie, des dermatites desquamantes ou en vue d'un traitement cosmétique.

Les dérivés pyridiniques de formule (I) peuvent être préparés notamment selon les méthodes décrites dans US-A-4 021 562 et US-A-4 080 500.

Les dérivés pyridiniques de formule (I) peuvent être préparés notamment selon les méthodes décrites dans US-A-3.910.928, US-A-4.032.559, EP-A-356.271, FR-89/11352, FR-90/01148 ou FR-90/06693.

Les dérivés triaziniques peuvent être préparés notamment selon les méthodes décrites dans US-A-3.475.340, US-A-3.270.014, US-A-3.270.018, US-A-3.270.015 ou FR-90/07664.

Comme acides convenables pour les sels d'addition par des acides des composés de formule (I), on peut citer notamment les acides chlorhydrique, bromhydrique, sulfurique, phosphorique, nitrique, acétique, benzoïque, salicylique, glycolique, succinique, nicotinique, tartrique, maléique, malique, palmoïque, méthane-sulfonique, cyclohexane-sulfamique, picrique, lactique et acéturique, notamment.

Dans la formule (I), alkyle inférieur représente les alkyles en C₁ à C₈, et notamment les radicaux méthyle, éthyle, n-propyle, isopropyle, n-butyle, isobutyle, méthyl-2 propyle, n-pentyle, n-hexyle, n-heptyle ou n-octyle.

Les radicaux alkyle en C₁ à C₁₈ sont choisis plus particulièrement parmi n-dodécyle, n-octyle, n-hexyle, n-butyle et éthyle.

Les radicaux alcényles désignent plus particulièrement allyle, 1-méthylallyle, 2-méthylallyle ou méthallyle, 2-butényle (crotyle), 3-butényle, 1,2-diméthylallyle, 1,1-diméthylallyle, 2-éthylallyle, 1-méthyl-2-butényle, 2-méthyl-2-butényle, 3-méthyl-2-butényle, 3-pentenyle, 2,3-diméthyl-2-butényle, 1,1,2-triméthylallyle, 1,3-diméthyl-2-butényle, 1-éthyl-2-butényle, 4-méthyl-2-pentényle, 2-éthyl-2-pentényle, 4,4-diméthyl-2-pentényle, 2-heptényle, 2-octényle, 5-octényle, 1,4-diméthyl-4-hexényle.

Par cycloalkyle, on désigne particulièrement cyclopropyle, 2-méthylcyclopropyle, 2,2-diméthcyclopropyle, 2,3-diéthylcyclopropropyle, 2-butylcyclopropyle, cyclobutyle, 2-méthylcyclobutyle, 3-propylcyclobutyle, 2,3,4-triéthylcyclobutyle, cyclopentyle, 2,2-diméthylcyclopentyle, 3-pentylcyclopentyle, 3-tert-butylcyclopentyle, cyclohexyle, 4-tert-butylcyclohexyle, 3-isopropylcyclohexyle, 2,2-diméthylcyclohexyle, cycloheptyle ou cyclooctyle .

Aryle signifie plus particulièrement phényle, 2,4-diméthyl phényle, 2,6-diméthoxyphényle ou 2,4-dichlorophényle.

Par aralkyle, on entend plus particulièrement benzyle, phénétyle, 1-phényléthyle, 2-phénylpropyle, 4-phénylbutyle, 5-phényl-2-méthylpentyle, 1-naphtylméthyle, 2-(1-naphtyl)éthyle ou 2-(2-naphtyl)-éthyle.

Parmi les composés de formule (I), on peut citer le minoxidil, le diamino-2,4 pipéridino-6 triazine oxyde-3, l'amino-2 méthyl-4 pipéridino-6 pyrimidine oxyde-3, le méthyl-2 amino-4 pipéridino-6 pyrimidine oxyde-3, le diméthyl-2,4 pipéridino-6 pyrimidine oxyde-3, le diamino-2,4-n-butyloxy-6 pyrimidine oxyde-3, le diamino-2,4-n-hexylamino-6 pyrimidine oxyde-3 et le diamino-2,6 pipéridino-4 pyridine oxyde-1.

Par agent de sulfatation tel qu'il est mis en oeuvre dans le composant b) selon l'invention, on entend les générateurs de SO₃ tels que décrits dans l'article de E. GILBERT, Chem. Rev. (1962), 62, 549-589, choisis par exemple parmi les complexes de SO₃ et d'amines tertiaires et plus particulièrement les complexes de SO₃ et d'amines mono- ou polyfonctionnelles, y compris les amines situées sur les groupements latéraux de chaînes polymériques. Selon l'invention, on utilise de préférence les complexes SO₃-triméthylamine et SO₃-triéthylamine.

Les agents ou "kits " selon l'invention sont destinés à transformer les composés de formule (I) en leur forme sulfo-conjuguée de formule (II), conformément au schéma ci-dessous : 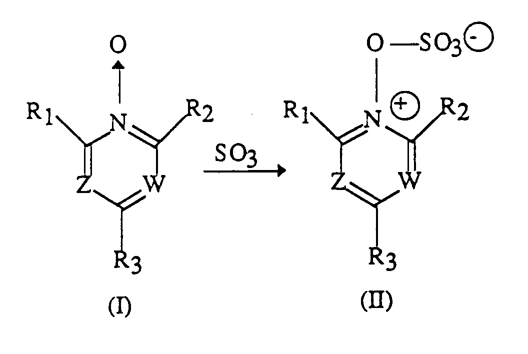
où les substituants R₁, R₂, R₃, Z et W ont les significations données ci-dessus, dans un milieu physiologiquement acceptable.

Une fois la réaction de sulfatation terminée, la forme sulfoconjuguée peut être, dans le milieu réactionnel, directement employée dans un traitement thérapeutique ou cosmétique. Ainsi, le milieu réactionnel peut servir de support d'application.

Pour obtenir le milieu réactionnel qui soit également support d'application, le dispositif selon l'invention peut se présenter sous la forme d'un récipient bi-compartimenté. Dans cette forme de réalisation, le premier compartiment contient le premier composant, de préférence sous forme de solution et le second compartient contient le second composant sous forme liquide ou solide, les contenus des deux compartiments étant destinés à être mélangés avant l'emploi pour former un milieu physiologiquement acceptable.

Les milieux physiologiquement acceptables peuvent être aqueux, hydroalcooliques ou anhydres.

Pour des préparations à usage thérapeutique buvables, on préfere particulièrement les milieux aqueux ou à faible teneur en alcool et pour les préparations à usage thérapeutique ou cosmétique par application topique, on peut prévoir des milieux hydroalcooliques, alcooliques ou anhydres.

Pour les préparations à usage topique, le milieu peut contenir également d'autres excipients tels que le propylèneglycol, les éthers de glycols ou de polyols, des agents épaississants, des stabilisants, des agents tensio-actifs, notamment.

Quelle que soit l'application, orale ou topique, à laquelle est destinée la préparation comportant la forme sulfo-conjuguée, le pH de la préparation, se situe autour de 7, et de préférence entre 5 et 9.

Selon l'invention, les concentrations en composés N-oxyde utilisés dépendent de l'activité du composé, de son innocuité et également de son mode d'administration. La concentration en forme sulfo-conjuguée de formule (II) est comprise entre O,1 et 10% en poids du mélange de tous les composants.

En ce qui conceme la quantité d'agents de sulfatation, celle-ci dépend de son activité et de la cinétique de la réaction et également du taux de sulfatation désiré dans la préparation. Dans le cas où l'on souhaite une grande rapidité ou un taux élevé de sulfatation, on utilisera de préférence un excès d'agent de sulfatation. La quantité d'agent de sulfatation compris dans le composant b) pouvant aller jusqu'à 100 équivalents molaire de la quantité de composé N-oxyde de formule (I) contenu dans le composant a).

La durée de réaction des composés de formule (I) avec le complexe SO₃-amines peut varier entre environ 5 minutes et environ 2 heures.

Selon l'invention, une forme particulière de réalisation est constituée par un agent ou kit comportant deux composants. Le premier composant (a) se présente sous forme d'une solution du composé de formule (I) dans un mélange alcoolique, hydroalcoolique ou aqueux.

Le second composant (b) se présente sous forme solide, par exemple sous forme d'une pastille composée contenant l'agent de sulfatation et un support solide acceptable.

Le second composant (b) peut en particulier se présenter sous forme d'un comprimé effervescent comprenant l'agent de sulfatation et un système générateur de gaz carbonique susceptible de provoquer l'effervescence, ce système étant constitué d'un acide choisi parmi les acides solides hydrosolubles tels que des acides aliphatiques comme l'acide acétique, propionique, butyrique, valérianique; des acides dicarboxyliques comme l'acide oxalique, malonique, succinique, glutarique, adipique, pimélique, fumarque, maléique, phtalique, isophtalique, térephtalique; des acides aminés tels que l'acide glutamique; des hydroxyacides tels que l'acide lactique, malique, tartrique, citrique ainsi que leurs sels.

Les acides peuvent également être choisis parmi les acides inorganiques tels que l'acide phosphorique et ses sels comme les sels acides de potassium, de sodium ou encore les sulfites, bisulfites de sodium ou de potassium ou d'ammonium.

Le second constituant du système effervescent est un carbonate tel qu'un carbonate ou hydrogénocarbonate de sodium, potassium ou d'ammonium.

Le rapport en poids de l'acide au carbonate est tel à provoquer l'effervescence et peut varier suivant la nature de l'acide et du carbonate. Généralement, le carbonate est présent dans des proportions de 5 à 80% en poids du système effervescent et l'acide dans des proportions de 20 à 85%.

La quantité de système effervescent par rapport au poids du compté étant comprise de préférence entre 50 et 80% en poids.

Avant l'emploi, on mélange le composant b) avec le composant a) sous forme de solution. Après un temps de réaction convenable, le composé de formule (I) initiale se présente totalement ou partiellement sous forme sulfo-conjuguée. Le produit réactionnel peut être alors appliqué directement sur les zones d'application topique ou peut être ingéré.

Il est possible d'améliorer la cinétique de la réaction du présent procédé par un apport de chaleur. Cet apport peut être réalisé de façon externe ou bien généré par réaction exothermique d'adjuvants.

Les dérivés sulfo-conjugués ainsi préparés peuvent présenter, dans le même milieu, une stabilité améliorée par rapport aux "sulfates" préparés par synthèse.

La présente invention concerne également un procédé de mise en oeuvre des agents selon l'invention, caractérisé en ce que l'on met en contact les composants a), b) et éventuellement c) définis ci-dessus, dans l'un des ordres suivants :
- a) est mis en contact avec b)
- a) est mélangé à c) et le mélange résultant a) + c) est mis en contact avec b)
- b) est mélangé à c) et le mélange résultant b) + c) est mis en contact avec a)
- a) est mis en contact avec b), le produit résultant étant mélangé à c).

La présente invention concerne aussi le procédé de préparation de la forme sulfo-conjuguée de composés pyridino-, pyrimidino- ou triazino-N-oxyde prêt à l'emploi, caractérisé en ce que :
a/ on stocke un composant comportant au moins un compost de formule (I) : 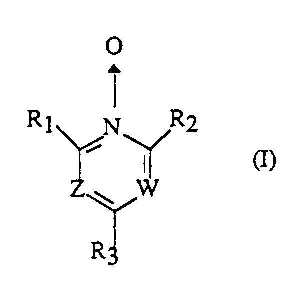 dans laquelle les substituants sont définis ci-dessus,
b/ on stocke un composant comportant au moins un agent de sulfatation, et
c/ on mélange les composants stockés.

L'invention concerne également l'utilisation des agents ou "kits" à plusieurs composants selon l'invention, pour la préparation de la forme sulfo-conjuguée de composés pyridino-, pyrimidino- ou triazino-N-oxyde destinés au traitement de l'hypertension ainsi que l'utlisation du dispositif pour la préparation de cette forme sulfo-conjuguée destiné au traitement de la chute des cheveux ou à la stimulation de leur repousse.

Les exemples suivants sont destinés à décrire l'invention, sans en limiter la portée.

### EXEMPLE 1

Dans un flacon unidose contenant 2 ml de solution à 50° alcoolique de Minoxidil à 1% et 100 mg de bicarbonate de sodium, on introduit 60 mg de complexe SO₃-N,N-diisopropyléthylamine en poudre conditionné sous atmosphère inerte. Après quelques minutes de dégagement gazeux, la solution prend une teinte orangée, et le pH se stabilise à 7.

Cinq minutes après le début du mélange, on réalise avec la solution une chromatographie en couche mince (CCM) sur plaque de silice. Après élution par un mélange acétate d'éthyle/méthanol (80/20), on observe en UV à 254 nm une seule tache correspondant au minoxidil 0-sulfate (R_{f}=0,88, R_{f} minoxidil = 0,15).

### EXEMPLE 2

Dans un flacon unidose, on conditionne 3 ml de minoxidil à 1% dans un mélange ternaire éthanol/propylèneglycol/eau (50/20/30). On ajoute 90 mg de complexe SO₃-triéthylamine en poudre conditionné sous atmosphère inerte. Après une légère agitation et 5 minutes de contact, le mélange devient homogène, et le pH se stabilise à 8.

Déposée sur plaque de silice et après élution par un mélange acétate d'éthyle/méthanol (80/20), la solution révèle en UV une tache importante correspondant au minoxidil 0-sulfate et une tache correspondant au minoxidil résiduel.

Abandonnée à température ambiante, la même solution après 50 minutes, ne montre plus en CCM qu'une tache correspondant au sulfate.

### EXEMPLE 3

On conditionne dans un flacon unidose 2 ml de minoxidil à 1% dans un mélange binaire éthanol absolu/propylèneglycol (95/5). On ajoute à cette solution 20 mg de complexe SO₃-triéthylamine en poudre conditionné sous atmosphère inerte.

Après une légère agitation et 10 minutes de contact, le mélange est devenu homogène. Après CCM sur plaque de silice et élution par un mélange acétate d'éthyle/méthanol (50/50), on observe en UV une tache très majoritaire correspondant au sulfate (R_{f}=0,9) et une légère tache correspondant au minoxidil (R_{f}=0,3).

### EXEMPLE 4

A 2 ml de minoxidil à 1% dans un mélange éthanol/eau (50/50), on ajoute un mélange de 100 mg de bicarbonate de sodium et de 150 mg de complexe SO₃-poly(4-vinylpyridine) à 2,2 mmoles SO₃/g.

Après un léger dégagement de CO₂ et 15 minutes de contact, on observe un léger dépôt dû au support polymérique insoluble. Le surnageant montre en CCM sur plaque de silice éluée par un mélange acétate d'éthyle/méthanol (50/50), une tache correspondant au minoxidil 0-sulfate.

### EXEMPLE 5

On réalise dans un mortier le mélange solide suivant :

| | |
|---|---|
| Acide citrique (à 1H₂0) | 10 g |
| Bicarbonate de sodium | 15 g |
| Complexe SO₃-triméthylamine | 5 g |

Après un broyage destiné à réaliser une poudre homogène, on conditionne sous atmosphère inerte par sachets de 0,3 g.

On conditionne par ailleurs une solution de minoxidil à 0,5 % dans un mélange hydroalcoolique à 35° alcoolique dans des flacons unidose à raison de 5 ml chacun.

Avant utilisation, on ajoute le contenu d'un sachet dans un flacon unidose. Après la cessation de l'effervescence, au bout de quelques minutes, la solution présente un pH de 6.

Par dosage en chromatographie liquide haute performance du sulfate de minoxidil formé, on observe un taux de sulfatation de 50% après 30 minutes de contact à température ambiante.

### EXEMPLE 6

On réalise dans un mortier le mélange solide suivant :

| | |
|---|---|
| Acide citrique (à 1H₂0) | 10 g |
| Bicarbonate de sodium pur | 15 g |
| Complexe SO₃-triméthylamine | 5 g |
| Silicagel 60 (230-400 Mesh) | 20 g |

Après broyage de l'ensemble jusqu'à obtention d'une poudre fine homogène, on conditionne sous atmosphère inerte par sachets de 0,5 g.

On conditionne par ailleurs le minoxidil par unités de 5 ml comme dans l'exemple 5.

Avant utilisation, on ajoute à un flacon unidose de minoxidil, le contenu d'un sachet. On adapte ensuite sur le flacon un embout applicateur en mousse.

Au cours de l'effervescence, le comprimé se désagrège et après quelques minutes, il ne subsiste plus qu'un léger dépôt de silice au fond du flacon, qui sera retenu par l'embout applicateur en mousse.

Par dosage en chromatographie liquide haute performance, on observe un taux de sulfatation du minoxidil de 50% après 30 minutes de contact.

### EXEMPLE 7

On réalise dans un mortier le mélange solide suivant :

| | |
|---|---|
| Acide salicylique pur | 20 g |
| Bicarbonate de sodium | 15 g |
| Complexe SO₃-triméthylamine | 5 g |
| Silicagel 60 (230-400 Mesh) | 10 g |

Après obtention d'une poudre homogène, on prépare à partir de ce mélange, des comprimés de 0,5 g que l'on conditionne par tablettes sous emballage plastifié. On effectue par ailleurs, un conditionnement du minoxidil en flacon unidose de 5 ml comme dans l'exemple 5.

Pour obtenir la préparation de sulfate, on ajoute un comprimé effervescent à un flacon uni-dose de minoxidil. Au cours de l'effervescence, le comprimé se désagrège et après quelques minutes, il ne subsiste plus qu'un léger dépôt de silice au fond du flacon, qui sera retenu par l'embout applicateur en mousse.

Après 30 minutes de contact, le dosage chromatographique indique un taux de sulfatation de 50%. L'abandon de la préparation à température ambiante durant plusieurs jours se traduit par un taux de sulfatation de 42% après 7 jours, avec un pH de 7,5.

Une composition ainsi préparée prèsente donc une stabilité supérieure par rapport à une composition préparée par dissolution du sulfate de minoxidil, pouvant ainsi convenir à un traitement durant une semaine en application quotidienne le matin et le soir.

### EXEMPLE 8

On mélange dans un mortier les ingrédients suivants :

| | |
|---|---|
| Acide salicylique | 20 g |
| Bicarbonate de potassium | 18 g |
| Complexe SO₃-triméthylamine | 10 g |
| Silicagel 60 (230-400 Mesh) | 10 g |

Après obtention d'une poudre homogène, on prépare des comprimés de 0,58 g que l'on conditionne en tablettes sous emballage protecteur.

En utilisant un conditionnement de minoxidil comme dans l'exemple 5 et en ajoutant un comprimé effervescent pour une dose de 5 ml de minoxidil, on obtient après 10 minutes un taux de sulfatation de 50%. Ce taux se maintient à 40% après abandon de la préparation durant 7 jours, à température ambiante.

### EXEMPLE 9

A un flacon unidose de 5 ml de solution à 0,5% dans un mélange hydro-alcoolique (à 35 % alcoolique) de diamino-2,4 pipéridino-6 triazine oxyde-3, on ajoute un comprimé effervescent préparé selon l'exemple 8.

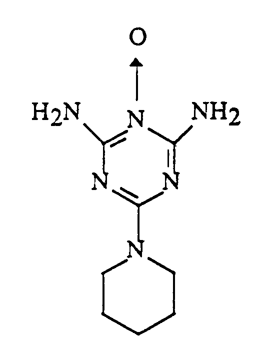

Après cessation de l'effervescence, on observe en CCM sur plaque de silice, l'apparition d'une tache de dérivé sulfo-conjugué après élution par le mélange acétate d'éthyle/méthanol (50/50).

| | |
|---|---|
| R_{f} du sulfo-conjugué | 0,93 |
| R_{f} du N-oxyde | 0,35 |

### EXEMPLE 10

On procède en tout point comme dans l'exemple 9, en utilisant comme N-oxyde le dérivé amino-2 méthyl-4 pipéridino-6 pyrimidine oxyde-3.

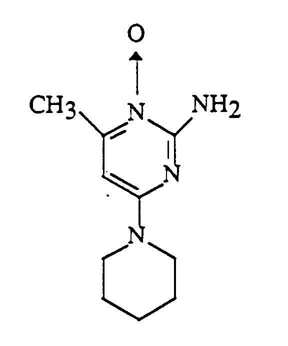

| | |
|---|---|
| R_{f} du sulfo-conjugué | 0,90 |
| R_{f} du N-oxyde | 0,38 |

### EXEMPLE 11

On procède comme dans l'exemple 9, en utilisant comme N-oxyde le dérivé méthyl-2 amino-4 pipéridino-6 pyrimidine oxyde-3.

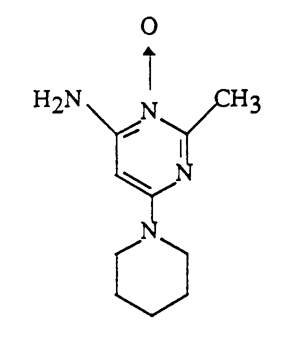

| | |
|---|---|
| R_{f} du sulfo-conjugué | 0,91 |
| R_{f} du N-oxyde | 0,41 |

### EXEMPLE 12

On procède comme dans l'exemple 9, avec le dérivé diméthyl-2,4 pipéridino-6 pyrimidine oxyde-3.

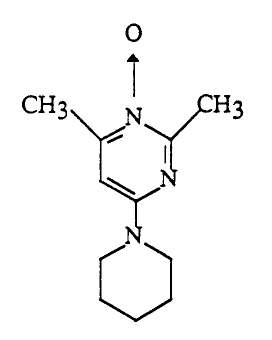

| | |
|---|---|
| R_{f} du sulfo-conjugué | 0,8 |
| R_{f} du N-oxyde | 0,41 |

### EXEMPLE 13

A un flacon uni-dose de 5 ml de solution à 0,2% dans un mélange hydro-alcoolique (à 35° alcoolique) de diamino-2,4-n-butyloxy-6 pyrimidine oxyde-3, on ajoute un compripmé effervescent préparé selon l'exemple 8.

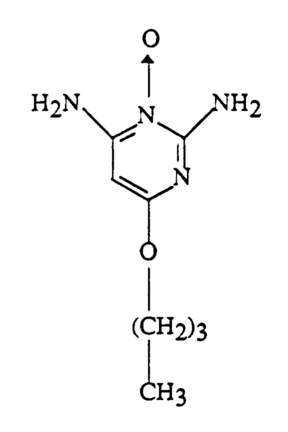

Après cessation de l'effervescence, on observe en CCM une tache de dérivé sulfo-conjugué.

### EXEMPLE 14

A un flacon uni-dose de 5 ml de solution à 0,5% dans un mélange hydro-alcoolique (à 35° alcoolique) de diamino-2,4 n-hexylamino-6 pyrimidine oxyde-3, on ajoute un comprimé effervescent préparé selon l'exemple 8.

Après cessation de l'effervescence, on observe en CCM une tache de dérivé sulfo-conjugué.

### EXEMPLE 15

A un flacon uni-dose de 5 ml de solution à 0,5% dans un mélange hydro-alcoolique (à 35° alcoolique) de diamino-2,6 pipéridino-4 pyridine oxyde-1, on ajoute un comprimé effervescent préparé selon l'exemple 8.

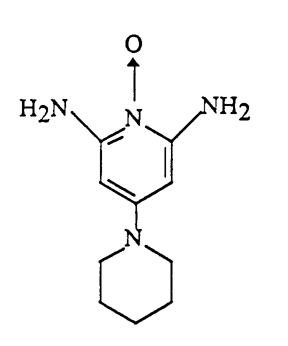

Après cessation de l'effervescence, on observe en CCM une tache de dérivé sulfo-conjugué.

## Revendications

1. Agent à plusieurs composants ou "kit" à plusieurs composants de préparation et de stabilisation de la forme sulfo-conjuguée de composés pyridino-, pyrimidino- ou triazino-N-oxyde, caractérisé en ce qu'il comporte sous forme séparée au moins :
a) un composant contenant dans un milieu alcoolique, hydroalcoolique ou aqueux au moins un composé de formule (I) : 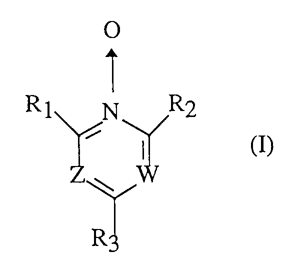 dans laquelle :
R₁ et R₂ désignent alkyle inférieur en C₁ à C₈ ou un groupement amino;
W et Z désignent chacun un groupement -CH= ou -N=;
R₃ désigne un groupement :
-S-R₄,-O-R₄, 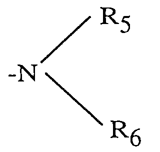 ou -H; et
R₄ désigne un groupement alkyle linéaire ou ramifié en C₁ à C₁₈ non substitué ou substitué par un ou plusieurs atomes d'halogène; alcényle en C₁ à C₁₈; cycloalkyle en C₃ à C₇; aryle ou aralkyle;
R₅ et R₆ désignent, indépendamment l'un de l'autre, un atome d'hydrogène ou un groupement tel que défini pour R₄; ou R₅ et R₆ forment avec l'azote auquel ils sont reliés, un reste hétérocyclique choisi dans le groupe constitué par les groupements pipéridino, pyrrolidinyle, morpholino, 2,4,4-triméthylazétidinyle, 2,3,4-triméthylazétidinyle, 2-méthylpyrrolidinyle, 3-butylpyrrolidinyle₁ 2-isohexylpyrrolidinyle, 2,3-diméthylpyrrolidinyle, 2,2-diméthylpyrrolidinyle, 2,5-diéthylpyrrolidinyle, 3-tert-butylpyrrolidinyle, 2,3,5-triméthylpyrrolidinyle, 3,4-dioctylpyrrolidinyle, 2-méthylpipéridino, 3-méthylpipéridino, 4-méthylpipéridino, 3-isopropylpipéridino, 4-tert-butylpipéridino, 2-méthyl-5-éthylpipéridino, 3,5-dipentylpipéridino, 2,4,6-triméthylpipéridino, 2,6-diméthylpipéridino, 2,6-diméthyl-4-octylpipéridino, 2,3,5-triéthylpipéridino, 2-éthylhexahydroazepinyle, 4-tert-butylhexahydroazépinyle, 3-heptylhexahydroazépinyle, 2,4-diméthylhexaliydroazépinyle, 3,3-diméthylhexahydroazépinyle, 2,4,6-tripropylhexahydroazépinyle, 2-méthylheptaméthylène-imino, 5-butylheptaméthylène-imino, 2,4-diisopropylheptaméthylène-imino, 3,3-diéthylheptaméthylène-imino, 2,5,8-triméthylheptaméthylène-imino, 3-méthyloctaméthylène-imino, 2,9-diéthyloctaméthylène-imino, 4-isooctyloctaméthylène-imino, 2-éthylmorpholino, 2-méthyl-5-éthylmorpholino, 3,3-diméthylmorpholino, 2,6-di-ter-butylmorpholino, 4-méthylpipérazinyle, 4-isopropylpipérazinyle, 2-méthylaziridinyle, 2-éthylaziridinyle, 2-butylaziridinyle, 2,3-diméthylaziridinyle, 2,2-diméthylaziridinyle, 2-méthylazétidinyle, 3-méthylazétidinyle, 2-octylazétidinyle, 2,2-diméthylazétidinyle, 3,3-diéthylazétidinyle; ou leurs sels d'addition par des acides; et
b) un composant comportant au moins un agent de sulfatation sous forme solide, les composants étant destinés à être mis en contact avant leur emploi pour le traitement de la chute des cheveux ou la stimulation de leur repousse.

2. Agent ou kit selon la revendication 1, caractérisé en ce qu'il comporte en outre un composant c) constitué par un milieu liquide, le composant c) étant destiné à être additionné au composant a) ou b) avant la mise en contact avec b) ou a) ou aux composants a) et b) après leur mise en contact.

3. Agent ou kit selon la revendication 1, caractérisé en ce que l'agent de sulfatation est choisi parmi les complexes SO₃-amines polymériques ou monomériques.

4. Agent ou kit selon l'une des revendications 1 à 4, caractérisé en ce que l'agent de sulfatation est choisi parmi les complexes de SO₃ et d'amines tertiaires.

5. Agent ou kit selon l'une des revendications 1 à 4, caractérisé en ce que l'agent de sulfatation est choisi parmi les complexes SO₃-triméthylamine et SO₃-triéthylamine.

6. Agent ou kit selon l'une des revendications 1 à 5, caractérisé en ce que le composant b) est soluble dans a) et/ou c).

7. Agent ou kit selon l'une des revendications 1 à 6, caractérisé en ce que le composant a) comporte 0,1 à 10% en poids du composé de formule (I) par rapport au poids total de a), b) et c).

8. Agent ou kit selon l'une des revendications 1 à 7, caractérisé en ce que le pH résultant de la mise en contact de a), b), c) est compris entre 5 et 9.

9. Agent ou kit selon l'une des revendications 1 à 8, caractérisé en ce que la concentration molaire en agent de sulfatation présent dans b), est comprise entre 1 et 100 fois la concentration molaire en composé de formule (I) présent dans a).

10. Agent selon la revendication 1, caractérisé par le fait que le composant (b) se présente sous forme d'un comprimé effervescent comprenant l'agent de sulfatation et un système générateur de gaz carbonique constitué d'un acide solide et hydrosoluble et d'un carbonate.

11. Agent ou kit selon l'une des revendications précédentes, caractérisé en ce que le composé de formule (I) est choisi parmi le minoxidil, le diamino-2,4 pipéridino-6 triazine oxyde-3, l'amino-2 méthyl-4 pipéridino-6 pyrimidine oxyde-3, le méthyl-2 amino-4 pipéridino-6 pyrimidine oxyde-3, le diméthyl-2,4 pipéridino-6 pyrimidine oxyde-3, le diamino-2,4-n-butyloxy-6 pyrimidine oxyde-3, le diamino-2,4-n-hexylamino-6 pyrimidine oxyde-3 et le diamino-2,6 pipéridino-4 pyridine oxyde-1.

12. Procédé de mise en oeuvre de l'agent ou kit selon l'une des revendications précédentes, caractérisé en ce que l'on met en contact les composants a), b) et c) définis dans la revendication 1 à 11, dans l'un des ordres suivants :
- a) est mis en contact avec b)
- a) est mélangé à c) et le mélange résultant a) + c) est mis en contact avec b)
- b) est mélangé à c) et le mélange résultant b) + c) est mis en contact avec a)
- a) est mis en contact avec b), le produit résultant étant mélangé à c).

13. Procédé de préparation de la forme sulfo-conjuguée de composés pyridino-, pyrimidino- ou triazino-N-oxyde prête à l'emploi, caractérisé en ce que :
a) on stocke un composant dans une solution aqueuse hydroalcoolique ou alcoolique comportant au moins un composé de formule (I) : 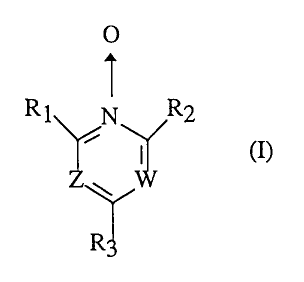 dans laquelle :
R₁ et R₂ désignent alkyle inférieur en C₁ à C₈ ou un groupement amino;
W et Z désignent chacun un groupement -CH= ou -N= ;
R₃ désigne un groupement :
-S-R₄,-O-R₄, 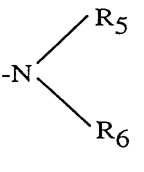 ou -H; et
R₄ désigne un groupement alkyle linéaire ou ramifié en C₁ à C₁₈ non substitué ou substitué par un ou plusieurs atomes d'halogène; alcényle en C₁ à C₁₈; cycloalkyle en C₃ à C₇; aryle ou aralkyle;
R₅ et R₆ désignent, indépendamment l'un de l'autre, un atome d'hydrogène ou un groupement tel que défini pour R₄; ou R₅ et R₆ forment avec l'azote auquel ils sont reliés, un reste hétérocyclique choisi dans le groupe constitué par les groupements pipéridino, pyrrolidinyle, morpholino, 2,4,4-triméthylazétidinyle, 2,3,4-triméthylazétidinyle, 2-méthylpyrrolidinyle, 3-butylpyrrolidinyle, 2-isohexylpyrrolidinyle, 2,3-diméthylpyrrolidinyle, 2,2-diméthylpyrrolidinyle, 2,5-diéthylpyrrolidinyle, 3-tert-butylpyrrolidinyle, 2,3,5-triméthylpyrrolidinyle, 3,4-dioctylpyrrolidinyle, 2-méthylpipéridino, 3-méthylpipéridino, 4-méthylpipéridino, 3-isopropylpipéridino, 4-tert-butylpipéridino, 2-méthyl-5-éthylpipéridino, 3,5-dipentylpipéridino, 2,4,6-triméthylpipéridino, 2,6-diméthylpipéridino, 2,6-diméthyl-4-octylpipéridino, 2,3,5-triéthylpipéridino, 2-éthylhexahydroazepinyle, 4-tert-butylhexahydroazépinyle, 3-heptylhexahydroazépinyle, 2,4-diméthylhexahydroazépinyle, 3,3-diméthylhexahydroazépinyle, 2,4,6-tripropylhexahydroazépinyle, 2-méthylheptaméthylène-imino, 5-butylheptaméthylène-imino, 2,4-diisopropylheptaméthylène-imino, 3,3-diéthylheptaméthylène-imino, 2,5,8-triméthylheptaméthylène-imino, 3-méthyloctaméthylène-imino, 2,9-diéthyloctaméthylène-imino, 4-isooctyloctaméthylène-imino, 2-éthylmorpholino, 2-méthyl-5-éthylmorpholino, 3,3-diméthylmorpholino, 2,6-di-ter-butylmorpholino, 4-méthylpipérazinyle, 4-isopropylpipérazinyle, 2-méthylaziridinyle, 2-éthylaziridinyle, 2-butylaziridinyle, 2,3-diméthylaziridinyle, 2,2-diméthylaziridinyle, 2-méthylazétidinyle, 3-méthylazétidinyle, 2-octylazétidinyle, 2,2-diméthylazétidinyle, 3,3-diéthylazétidinyle; ou leurs sels d'addition par des acides;
b) on stocke un composant comportant au moins un agent de sulfatation sous forme solide; et
c) on mélange les composants stockés.

## Claims

1. Multicomponent agent or multicomponent "kit" for preparing and stabilising the sulpho-conjugated form of pyridino, pyrimidino or triazino N-oxide compounds, characterised in that they comprise, in separated form, at least:
a) a component containing, in an alcoholic, aqueous-alcoholic or aqueous medium, at least one compound of formula (I): 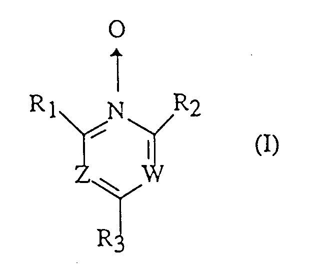 in which:
R₁ and R₂ denote a C₁ to C₈ lower alkyl or an amino group;
W and Z each denote a -CH= or -N= group;
R₃ denotes a
-S-R₄,-O-R₄, 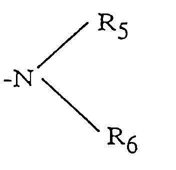 or -H group; and
R₄ denotes a linear or branched C₁ to C₁₈ alkyl group which is unsubstituted or substituted by one or more halogen atoms, a C₁ to C₁₈ alkenyl, C₃ to C₇ cycloalkyl, aryl or aralkyl group;
R₅ and R₆ denote, independently of each other, a hydrogen atom or a group such as defined for R₄; or R₅ and R₆ form with the nitrogen to which they are attached, a heterocyclic residue chosen from the group consisting of piperidino, pyrrolidinyl, morpholino, 2,4,4-trimethylazetidinyl, 2,3,4-trimethylazetidinyl, 2-methylpyrrolidinyl, 3-butylpyrrolidinyl, 2-isohexylpyrrolidinyl, 2,3-dimethylpyrrolidinyl, 2,2-dimethylpyrrolidinyl, 2,5-diethylpyrrolidinyl, 3-tert-butylpyrrolidinyl, 2,3,5-trimethylpyrrolidinyl, 3,4-dioctylpyrrolidinyl, 2-methylpiperidino, 3-methylpiperidino, 4-methylpiperidino, 3-isopropylpiperidino, 4-tert-butylpiperidino, 2-methyl-5-ethylpiperidino, 3,5-dipentylpiperidino, 2,4,6-trimethylpiperidino, 2,6-dimethylpiperidino, 2,6-dimethyl-4-octylpiperidino, 2,3,5-trimethylpiperidino, 2-ethylhexahydroazepinyl, 4-tert-butylhexahydroazepinyl, 3-heptylhexahydroazepinyl, 2,4-dimethylhexahydroazepinyl, 3,3-dimethylhexahydroazepinyl, 2,4,6-tripropylhexahydroazepinyl, 2-methylheptamethylene-imino, 5-butylheptamethylene-imino, 2,4-diisopropylheptamethylene-imino, 3,3-diethylheptamethylene-imino, 2,5,8-trimethylheptamethylene-imino, 3-methyloctamethylene-imino, 2,9-diethyloctamethylene-imino, 4-isooctyloctamethylene-imino, 2-ethylmorpholino, 2-methyl-5-ethylmorpholino, 3,3-dimethylmorpholino, 2,6-di-tert-butylmorpholino, 4-methylpiperazinyl, 4-isopropylpiperazinyl, 2-methylaziridinyl, 2-ethylaziridinyl, 2-butylaziridinyl, 2,3-dimethylaziridinyl, 2,2-dimethylaziridinyl, 2-methylazetidinyl, 3-methylazetidinyl, 2-octylazetidinyl, 2,2-dimethylazetidinyl and 3,3-diethylazetidinyl groups; or their addition salts with acids; and
b) a component comprising at least one sulphating agent in solid form, the components being intended to be brought into contact before their use for the treatment of hair loss or for stimulating its regrowth.

2. Agent or kit according to Claim 1, characterised in that it furthermore comprises a component c) consisting of a liquid medium, the component c) being intended to be added to the component a) or b) before being brought into contact with b) or a), or to the components a) and b) after they have been brought into contact.

3. Agent or kit according to Claim 1, characterised in that the sulphating agent is chosen from the polymeric or monomeric SO₃-amine complexes.

4. Agent or kit according to one of Claims 1 to 4, characterised in that the sulphating agent is chosen from the SO₃ and tertiary amine complexes.

5. Agent or kit according to one of Claims 1 to 4, characterised in that the sulphating agent is chosen from the SO₃-trimethylamine and SO₃-triethylamine complexes.

6. Agent or kit according to one of Claims 1 to 5, characterised in that the component b) is soluble in a) and/or c).

7. Agent or kit according to one of Claims 1 to 6, characterised in that the component a) comprises 0.1 to 10% by weight of the compound of formula (I) relative to the total weight of a), b) and c).

8. Agent or kit according to one of Claims 1 to 7, characterised in that the pH resulting from the bringing into contact of a), b) and c) is between 5 and 9.

9. Agent or kit according to one of Claims 1 to 8, characterised in that the molar concentration of sulphating agent present in b) is between 1 and 100 times the molar concentration of the compound of the formula (I) present in a).

10. Agent according to Claim 1, characterised in that the component (b) is provided in the form of an effervescent tablet comprising the sulphating agent and a carbon dioxide gas generating system consisting of a solid and water soluble acid and a carbonate.

11. Agent or kit according to one of the preceding claims, characterised in that the compound of formula (I) is chosen from minoxidil, 2,4-diamino-6-piperidinotriazine 3-oxide, 2-amino-4-methyl-6-piperidinopyrimidine 3-oxide, 2-methyl-4-amino-6-piperidinopyrimidine 3-oxide, 2,4-dimethyl-6-piperidinopyrimidine 3-oxide, 2,4-diamino-6-n-butyloxypyrimidine 3-oxide, 2,4-diamino-6-n-hexylaminopyrimidine 3-oxide and 2,6-diamino-4-piperidinopyridine 1-oxide.

12. Method for using the agent or kit according to one of the preceding claims, characterised in that the components a), b) and c) defined in Claims 1 to 11, are brought into contact in one of the following orders:
- a) is brought into contact with b)
- a) is mixed with c) and the resulting mixture a) + c) is brought into contact with b)
- b) is mixed with c) and the resulting mixture b) + c) is brought into contact with a)
- a) is brought into contact with b), the resulting product being mixed with c).

13. Method for preparing the ready-to-use sulpho-conjugated form of pyridino, pyrimidino or triazino N-oxide compounds, characterised in that:
a) a component is stored in an aqueous, aqueous-alcoholic or alcoholic solution which comprises at least one compound of formula (I): 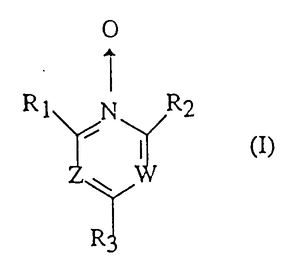 in which:
R₁ and R₂ denote a C₁ to C₈ lower alkyl or an amino group;
W and Z each denote a -CH= or -N= group;
R₃ denotes a
-S-R₄,-O-R₄, 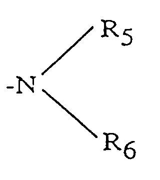 or -H group; and
R₄ denotes a linear or branched C₁ to C₁₈ alkyl group which is unsubstituted or substituted by one or more halogen atoms, a C₁ to C₁₈ alkenyl, C₃ to C₇ cycloalkyl, aryl or aralkyl group;
R₅ and R₆ denote, independently of each other, a hydrogen atom or a group such as defined for R₄; or R₅ and R₆ form with the nitrogen to which they are attached, a heterocyclic residue chosen from the group consisting of piperidino, pyrrolidinyl, morpholino, 2,4,4-trimethylazetidinyl, 2,3,4-trimethylazetidinyl, 2-methylpyrrolidinyl, 3-butylpyrrolidinyl, 2-isohexylpyrrolidinyl, 2,3-dimethylpyrrolidinyl, 2,2-dimethylpyrrolidinyl, 2,5-diethylpyrrolidinyl, 3-tert-butylpyrrolidinyl, 2,3,5-trimethylpyrrolidinyl, 3,4-dioctylpyrrolidinyl, 2-methylpiperidino, 3-methylpiperidino, 4-methylpiperidino, 3-isopropylpiperidino, 4-tert-butylpiperidino, 2-methyl-5-ethylpiperidino, 3,5-dipentylpiperidino, 2,4,6-trimethylpiperidino, 2,6-dimethylpiperidino, 2,6-dimethyl-4-octylpiperidino, 2,3,5-trimethylpiperidino, 2-ethylhexahydroazepinyl, 4-tert-butylhexahydroazepinyl, 3-heptylhexahydroazepinyl, 2,4-dimethylhexahydroazepinyl, 3,3-dimethylhexahydroazepinyl, 2,4,6-tripropylhexahydroazepinyl, 2-methylheptamethylene-imino, 5-butylheptamethylene-imino, 2,4-diisopropylheptamethylene-imino, 3,3-diethylheptamethylene-imino, 2,5,8-trimethylheptamethylene-imino, 3-methyloctamethylene-imino, 2,9-diethyloctamethylene-imino, 4-isooctyloctamethylene-imino, 2-ethylmorpholino, 2-methyl-5-ethylmorpholino, 3,3-dimethylmorpholino, 2,6-di-tert-butylmorpholino, 4-methylpiperazinyl, 4-isopropylpiperazinyl, 2-methylaziridinyl, 2-ethylaziridinyl, 2-butylaziridinyl, 2,3-dimethylaziridinyl, 2,2-dimethylaziridinyl, 2-methylazetidinyl, 3-methylazetidinyl, 2-octylazetidinyl, 2,2-dimethylazetidinyl and 3,3-diethylazetidinyl groups; or their addition salts with acids;
b) a component is stored which comprises at least one sulphating agent in solid form; and
c) the stored components are mixed.

## Patentansprüche

1. Mittel aus mehreren Bestandteilen oder "Kit" aus mehreren Bestandteilen zur Herstellung und zur Stabilisierung der Sulfo-konjugierten Form von Pyridin-, Pyrimidin- oder Triazin-N-oxid-Verbindungen,
dadurch **gekennzeichnet**, daß
sie in getrennter Form mindestens umfassen:
a) einen Bestandteil, enthaltend in einem alkoholischen, hydroalkoholischen oder wässrigen Milieu mindestens eine Verbindung der Formel (I): 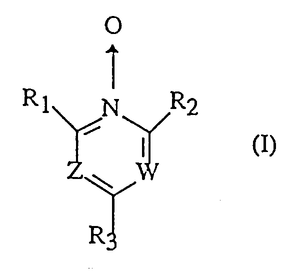 worin gilt:
R₁ und R₂ bedeuten einen C₁₋₈-Niedrigalkylrest oder eine Aminogruppe;
W und Z bedeuten jeweils eine Gruppierung -CH= oder -N=;
R₃ bedeutet eine Gruppierung:
-S-R₄, -O-R₄, 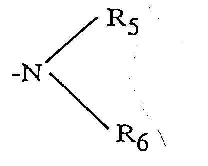 oder -H; und
R₄ bedeutet eine lineare oder verzweigte C₁₋₁₈-Alkylgruppe, die gegebenenfalls mit einem oder mehreren Halogenatomen substituiert ist, eine C₁₋₁₈-Akenyl-, C₃₋₇-Cycloalkyl-, Aryl- oder eine Aralkylgruppe;
R₅ und R₆ bedeuten, unabhängig voneinander, ein Wasserstoffatom oder eine für R₄ definierte Gruppe, oder R₅ und R₆ bilden mit dem Stickstoffatom, an das sie gebunden sind, einen heterozyklischen Rest, ausgewählt aus der Gruppe aus Piperidino-, Pyrrolidinyl-, Morpholino-, 2,4,4-Trimethylazetidinyl-, 2,3,4-Trimethylazetidinyl-, 2-Methylpyrrolidinyl-, 3-Butylpyrrolidinyl-, 2-Isohexylpyrrolidinyl-, 2,3-Dimethylpyrrolidinyl-, 2,2-Dimethylpyrrolidinyl-, 2,5-Diethylpyrrolidinyl-, 3-t-Butylpyrrolidinyl-, 2,3,5-Trimethylpyrrolidinyl-, 3,4-Dioctylpyrrolidinyl-, 2-Methylpiperidino-, 3-Methylpiperidino-, 4-Methylpiperidino-, 3-Isopropylpiperidino-, 4-t-Butylpiperidino-, 2-Methyl-5-ethylpiperidino-, 3,5-Dipentylpiperidino-, 2,4,6-Trimethylpiperidino-, 2,6-Dimethylpiperidino-, 2,6-Dimethyl-4-octylpiperidino-, 2,3,5-Triethylpiperidino-, 2-Ethylhexahydroazepinyl-, 4-t-Butylhexahydroazepinyl-, 3-Heptylhexahydroazepinyl-, 2,4-Dimethylhexahydroazepinyl-, 3,3-Dimethylhexahydroazepinyl-, 2,4,6-Tripropylhexahydroazepinyl-, 2-Methylheptamethylenimino-, 5-Butylheptamethylenimino-, 2,4-Diisopropylheptamethylenimino-, 3,3-Diethylheptamethylenimino-, 2,5,8-Trimethylheptamethylenimino-, 3-Methyloctamethylenimino-, 2,9-Diethyloctamethylenimino-, 4-Isooctyloctamethylenimino-, 2-Ethylmorpholino-, 2-Methyl-5-ethylmorpholino-, 3,3-Dimethylmorpholino-, 2,6-Di-t-butylmorpholino-, 4-Methylpiperazinyl-, 4-Isopropylpiperazinyl-, 2-Methylaziridinyl-, 2-Ethylaziridinyl-, 2-Butylaziridinyl-, 2,3-Dimethylaziridinyl-, 2,2-Dimethylaziridinyl-, 2-Methylazetidinyl-, 3-Methylazetidinyl-, 2-Octylazetidinyl-, 2,2-Dimethylazetidinyl- und aus 3,3-Diethylazedidinyl-Gruppierungen oder aus deren Säureadditionssalzen; und
b) einen Bestandteil, umfassend mindestens ein Sulfatierungsmittel in fester Form,
wobei die Bestandteile dazu bestimmt sind, vor deren Anwendung zur Behandlung des Ausfalls der Haare oder zur Anregung von deren erneutem Wachstum in Kontakt gebracht zu werden.

2. Mittel oder Kit gemäß Anspruch 1,
dadurch **gekennzeichnet**, daß
sie ausserdem einen Bestandteil c) aus einem flüssigen Milieu umfassen, wobei der Bestandteil c) dazu bestimmt ist, dem Bestandteil a) oder b) vor dem Zusmamenbingen mit b) oder a) oder den Bestandteilen a) und b) vor deren Zusammenbringung zugefügt zu werden.

3. Mittel oder Kit gemäß Anspruch 1,
dadurch **gekennzeichnet**, daß
das Sulfatiermittel aus polymeren oder monomeren SO₃-Amin-Komplexen ausgewählt ist.

4. Mittel oder Kit gemäß einem der Ansprüche 1 bis 3,
dadurch **gekennzeichnet**, daß
das Sulfatiermittel aus Komplexen von SO₃ und tertiären Aminen ausgewählt ist.

5. Mittel oder Kit gemäß einem der Ansprüche 1 bis 4,
dadurch **gekennzeichnet**, daß
das Sulfatiermittel aus SO₃-Trimethylamin- und SO₃-Triethylamin-Komplexen ausgewählt ist.

6. Mittel oder Kit gemäß einem der Ansprüche 1 bis 5,
dadurch **gekennzeichnet**, daß
der Bestandteil b) in a) und/oder c) löslich ist.

7. Mittel oder Kit gemäß einem der Ansprüche 1 bis 6,
dadurch **gekennzeichnet**, daß
der Bestandteil a) 0,1 bis 10 Gew.% Verbindung der Formel (I) enthält, bezogen auf das Gesamtgewicht von a), b) und c).

8. Mittel oder Kit gemäß einem der Ansprüche 1 bis 7,
dadurch **gekennzeichnet**, daß
der pH-Wert , der sich aus der Zusammenbringung von a), b) und c) einstellt, 5 bis 9 beträgt.

9. Mittel oder Kit gemäß einem der Ansprüche 1 bis 8,
dadurch **gekennzeichnet**, daß
die molare Konzentration an Sulfatiermittel, das in b) vorhanden ist, das 1- bis 100-fache der molaaren Konzentration an Verbindung der Formel (I) ausmacht, die in a) vorhanden ist.

10. Mittel gemäß Anspruch 1,
dadurch **gekennzeichnet**, daß
der Bestandteil b) in Form einer Brausetablette vorliegt, die das Sulfatiermittel und ein Erzeugungssystem für Kohlensäuregas enthält, welches aus einer festen und wasserlöslichen Säure und einem Carbonat zusammengesetzt ist.

11. Mittel oder Kit gemäß einem der vorhergehenden Ansprüche,
dadurch **gekennzeichnet**, daß
die Verbindung der Formel (I) aus Minoxidil, 2,4-Diamino-6-piperiddinoriazin-3-oxid, 2-Amino-4-methyl-6-piperidinopyrimidin-3-oxid, 2-Methyl-4-amino-6-piperidinopyrimidin-3-oxid, 2,4-Dimethyl-6-piperidinopyrimidin-3-oxid, 2,4-Diamino-6-n-butyloxipyrimidin-3-oxid, 2,4-Diamino-6-n-hexylaminopyrimidin-3-oxid und aus 2,6-Diamino-4-piperidinopyridin-1-oxid ausgewählt ist.

12. Verfahren zur Anwendung des Mittels oder Kit gemäß einem der vorhergehenden Ansprüche,
dadurch **gekennzeichnet**, daß
man die in Anspruch 1 bis 11 definierten Bestandteile a), b) und c) in einer der folgenden Reihenfolgen in Kontakt bringt:
- a) wird mit b) in Kontakt gebracht,
- a) wird mit c) vermischt, und die Mischung aus a) + c) wird mit b) in Kontakt gebracht,
- b) wird mit c) vermischt, und die Mischung aus b) + c) wird mit a) in Kontakt gebracht,
- a) wird mit b) in Kontakt gebracht, wobei das entstandene Produkt mit c) vermischt wird.

13. Verfahren zur Herstellung der sulfo-konjugierten Form von Pyridin-, Pyrimidin- oder Triazin-N-oxid-Verbindungen, die gebrauchsfertig vorliegen, wobei das Verfahren dadurch **gekennzeichnet** ist, daß man:
a) einen Bestandteil in einer wässrigen, hydroalkoholischen oder alkoholischen Lösung lagert und aufbewahrt, der mindestens eine Verbindung der Formel (I) enthält: 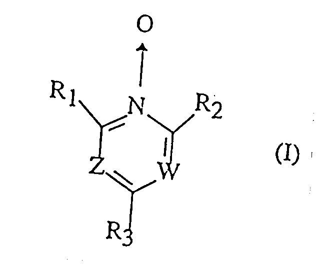 worin gilt:
R₁ und R₂ bedeuten einen C₁₋₈-Niedrigalkylrest oder eine Aminogruppe;
W und Z bedeuten jeweils eine Gruppierung -CH= oder -N=;
R₃ bedeutet eine Gruppierung:
-S-R₄, -O-R₄, 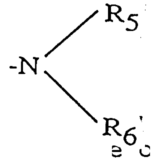 oder -H; und
R₄ bedeutet eine linear oder verzweigte C₁₋₁₈-Alkylgruppe, die gegebenenfalls mit einem oder mehreren Halogenatomen substituiert ist, eine C₁₋₁₈-Akenyl-, C₃₋₇-Cycloalkyl-, Aryl- oder eine Aralkylgruppe;
R₅ und R₆ bedeuten, unabhängig voneinander, ein Wasserstoffatom oder eine für R₄ definierte Gruppe, oder R₅ und R₆ bilden mit dem Stickstoffatom, an das sie gebunden sind, einen heterozyklischen Rest, ausgewählt aus der Gruppe aus Piperidino-, Pyrrolidinyl-, Morpholino-, 2,4,4-Trimethylazetidinyl-, 2,3,4-Trimethylazetidinyl-, 2-Methylpyrrolidinyl-, 3-Butylpyrrolidinyl-, 2-Isohexylpyrrolidinyl-, 2,3-Dimethylpyrrolidinyl-, 2,2-Dimethylpyrrolidinyl-, 2,5-Diethylpyrrolidinyl-, 3-t-Butylpyrrolidinyl-, 2,3,5-Trimethylpyrrolidinyl-, 3,4-Dioctylpyrrolidinyl-, 2-Methylpiperidino-, 3-Methylpiperidino-, 4-Methylpiperidino-, 3-Isopropylpiperidino-, 4-t-Butylpiperidino-, 2-Methyl-5-ethylpiperidino-, 3,5-Dipentylpiperidino-, 2,4,6-Trimethylpiperidino-, 2,6-Dimethylpiperidino-, 2,6-Dimethyl-4-octylpiperidino-, 2,3,5-Triethylpiperidino-, 2-Ethylhexahydroazepinyl-, 4-t-Butylhexahydroazepinyl-, 3-Heptylhexahydroazepinyl-, 2,4-Dimethylhexahydroazepinyl-, 3,3-Dimethylhexahydroazepinyl-, 2,4,6-Tripropylhexahydroazepinyl-, 2-Methylheptamethylenimino-, 5-Butylheptamethylenimino-, 2,4-Diisopropylheptamethylenimino-, 3,3-Diethylheptamethylenimino-, 2,5,8-Trimethylheptamethylenimino-, 3-Methyloctamethylenimino-, 2,9-Diethyloctamethylenimino-, 4-Isooctyloctamethylenimino-, 2-Ethylmorpholino-, 2-Methyl-5-ethylmorpholino-, 3,3-Dimethylmorpholino-, 2,6-Di-t-butylmorpholino-, 4-Methylpiperazinyl-, 4-Isopropylpiperazinyl-, 2-Methylaziridinyl-, 2-Ethylaziridinyl-, 2-Butylaziridinyl-, 2,3-Dimethylaziridinyl-, 2,2-Dimethylaziridinyl-, 2-Methylazetidinyl-, 3-Methylazetidinyl-, 2-Octylazetidinyl-, 2,2-Dimethylazetidinyl- und aus 3,3-Diethylazedidinyl-Gruppierungen, oder aus deren Säureadditionssalzen,
b) man einen Bestandteil lagert und aufbewahrt, der mindestens ein Sulfatiermittel in fester Form enthält, und
c) die gelagerten und aufbewahrten Bestandteile vermischt.
